# DEMANDE DE BREVET EUROPEEN

(11) **EP 2 065 028 A2**
(43) Date de publication de la demande: **03.06.2009**
(21) Numéro de dépôt: 08170208.6
(22) Date de dépôt: 28.11.2008
(51) Int. Cl.: A61K 8/58, A61K 8/81, A61K 8/891, A61K 8/898, A61Q 5/02, A61Q 5/06

(54) **Composition de coiffage repositionnable comprenant au moins un copolymère (méth)acrylique et au moins une silicone**

(30) Priorité: 30.11.2007 FR 0759475
(71) Demandeur: L'Oreal, 75008 Paris (FR)
(72) Inventeur: Decoster, Sandrine, 95210 Saint-Gratien (FR); Proust, Jean-Mary, 78410 Aubergenville (FR)
(74) Mandataire: Casalonga, Axel

(57) **Abrégé**

La présente invention concerne une composition de coiffage repositionnable des fibres kératiniques, et plus particulièrement des cheveux, caractérisée en ce qu'elle comprend dans un véhicule cosmétiquement acceptable :
(i) un ou plusieurs copolymères (méth)acrylique comprenant :
- (a) des motifs issus d'un ou de plusieurs monomères choisis parmi les monomères de (méth)acrylate de butyle, le ou les monomères (a) représentant de 15 à 50 % en poids par rapport à la quantité totale de monomères,
- (b) des motifs issus d'un ou de plusieurs monomères choisis parmi les monomères de (méth)acrylate d'hydroxyalkyle, et
- (c) des motifs éventuels issus d'un ou plusieurs monomères copolymérisables autres que lesdits monomères (a) et (b) ;

(ii) une ou plusieurs silicones.

La présente invention concerne également un procédé de traitement cosmétique la mettant en oeuvre et son utilisation comme shampoing ou après-shampooing.

## Description

La présente invention concerne une composition de coiffage repositionnable des fibres kératiniques, en particulier des fibres kératiniques humaines telles que les cheveux, comprenant un ou plusieurs copolymères (méth)acryliques et une ou plusieurs silicones.

L'invention concerne également un procédé de traitement des fibres kératiniques mettant en oeuvre ladite composition de coiffage et son utilisation comme shampooing ou après-shampooing.

Dans le domaine capillaire, la fixation de la coiffure est généralement un élément important du coiffage, et implique par exemple le maintien d'une forme qui a déjà été réalisée ou la mise en forme et la fixation simultanées des cheveux. Conformément à l'invention, le terme « composition de coiffage » concerne tout type de composition capillaire pouvant être utilisé pour effectuer le coiffage.

Les compositions de coiffage les plus courantes sur le marché des produits cosmétiques pour la mise en forme et/ou le maintien de la coiffure sont des compositions de spray comprenant une solution, habituellement à base d'alcool ou d'eau, et un ou plusieurs composés, généralement des résines polymères. L'une des fonctions des résines polymères est de former des liens entre les cheveux. Ces matières, également appelées fixateurs, se trouvent souvent en mélange avec différents adjuvants cosmétiques. Cette solution est généralement conditionnée soit dans un récipient aérosol approprié, qui est pressurisé à l'aide d'un propulseur, soit dans un flacon pompe.

Les compositions de coiffage peuvent également se présenter sous la forme de gels ou de mousses capillaires, qui sont généralement appliquées sur les cheveux mouillés avant d'effectuer un brushing ou un séchage.

Un inconvénient lié à l'utilisation de ces compositions de coiffage réside dans le fait qu'elles ne sont pas destinées à permettre à la coiffure d'être modifiée ultérieurement en une forme recherchée, qui est différente de celle initialement formée, sans recommencer de nouveau les opérations de coiffage et de fixation. Ceci est d'autant plus incommodant que, sous différents types de stress, la coiffure a tendance à prendre une forme permanente indésirable qui ne peut pas être facilement modifiée.

De plus, il peut s'avérer particulièrement intéressant pour un utilisateur que ces compositions de coiffage permettent également de conditionner les cheveux de manière satisfaisante, notamment en leur apportant de la douceur au toucher et en améliorant leur aptitude au peignage.

Par ailleurs, il est bien connu que, parmi les compositions de coiffage, les compositions rincées, telles que les shampooings ou les après-shampooings, ne permettent généralement pas de conférer un effet de coiffage aussi prononcé que celui obtenu avec des compositions non rincées telles que des gels ou des mousses. En particulier, on observe que l'effet de coiffage qui est obtenu à l'aide d'une composition rincée se traduit par un manque de tenue le jour même de l'application et dans le temps.

Ainsi, pour remédier à l'ensemble des inconvénients mentionnés ci-avant, la demande de brevet EP-A-1 321 130 décrit notamment des compositions rincées comprenant un ou plusieurs copolymères (méth)acryliques particuliers qui permettent de conférer aux cheveux un effet de coiffage dit « repositionnable » ou de recoiffage, c'est-à-dire que ces compositions apportent un effet de coiffage pouvant être restauré ou modifié sans avoir à recommencer de nouveau les opérations de coiffage et de fixation.

Cependant, de telles compositions de coiffage confèrent aux cheveux des propriétés cosmétiques insuffisantes, notamment en termes de douceur, de démêlage ou de brillance.

Par ailleurs, la facilité de coiffage n'est pas totalement satisfaisante.

Il existe donc un réel besoin de mettre au point des compositions de coiffage, de préférence des compositions rincées, telles que des shampooings ou après-shampooings, ne présentant pas l'ensemble des inconvénients décrits ci-dessus, c'est-à-dire qui permettent de conférer aux cheveux des propriétés cosmétiques satisfaisantes, notamment en termes de douceur, de démêlage tout en obtenant un effet de recoiffage prononcé et une bonne facilité de coiffage.

La demanderesse a maintenant découvert, de façon surprenante, qu'il était possible de formuler des compositions de coiffage ayant les propriétés recherchées, comprenant un ou plusieurs copolymères (méth)acryliques tels que définis ci-après (i) et une ou plusieurs silicones (ii).

La composition selon l'invention fournit un effet de coiffage repositionnable satisfaisant et est de préférence une composition rincée.

Par composition de coiffage « repositionnable », on entend une composition donnant un coiffage qui peut être restauré ou modifié sans appliquer à nouveau une matière ou une chaleur supplémentaire. Par exemple, pour restaurer ou modifier la coiffure dans le cas « d'alourdissement » ou de perte de mise en pli (décoiffage), on n'a besoin d'aucune nouvelle matière telle que l'eau ou une forme quelconque d'agent de fixation, ni de chaleur supplémentaire. L'efficacité de la composition peut être durable conduisant à un effet coiffant durable. D'autres termes pouvant être synonymes de repositionnable incluent redéformable, remoulable, recoiffable, ré-arrangeable et remodelable.

La composition de coiffage selon l'invention présente également l'avantage de conférer aux cheveux des propriétés cosmétiques satisfaisantes, notamment en termes de douceur, d'aptitude au démêlage et de brillance.

De plus, la composition de coiffage selon l'invention permet de faciliter aussi bien le coiffage que le recoiffage dans le cas où l'on souhaite modifier la coiffure sans avoir à appliquer une matière ou une chaleur supplémentaire.

La présente invention a donc notamment pour objet une composition de coiffage repositionnable, de préférence rincée, des fibres kératiniques, en particulier des fibres kératiniques humaines telles que les cheveux, comprenant, dans un véhicule cosmétiquement acceptable :
- (i) un ou plusieurs copolymères (méth)acrylique comprenant :
   (a) des motifs issus d'un ou plusieurs monomères choisis parmi les monomères de (méth)acrylate de butyle, le ou les monomères

   (a) représentant de 15 à 50 % en poids par rapport à la quantité totale de monomères,
   (b) des motifs issus d'un ou plusieurs monomères choisis parmi les monomères de (méth)acrylate d'hydroxyalkyle, et
   (c) des motifs éventuels issus d'un ou plusieurs monomères copolymérisables autres que lesdits monomères (a) et (b) ; et
- (ii) une ou plusieurs silicones.

La présente invention concerne également un procédé de traitement cosmétique des cheveux, comprenant l'application sur les cheveux de ladite composition de coiffage comprenant un ou plusieurs copolymères (méth)acryliques tels que définis ci-dessus (i) et une ou plusieurs silicones (ii).

Elle a aussi pour objet un procédé de traitement des cheveux, comprenant : (1) l'application sur les cheveux, avant la mise en forme initiale de la coiffure, d'une composition comprenant un ou plusieurs copolymères (méth)acryliques tels que décrit ci-dessus (i) et une ou plusieurs silicones (ii), ladite composition fournissant un effet repositionnable et étant de préférence une composition rincée telle qu'un shampoing ou un conditionneur, puis (2) la mise en forme de la coiffure au moins une fois, sans avoir besoin de composition ou de chaleur supplémentaire.

Elle concerne aussi l'utilisation de la composition selon l'invention comme shampoing ou après-shampooing.

D'autres objets et caractéristiques, aspects et avantages de l'invention apparaîtront encore plus clairement à la lecture de la description et des exemples qui suivent.

Le ou les copolymères (méth)acryliques (i) utilisés dans la composition selon l'invention, et leur procédé de fabrication, sont notamment décrits dans la demande de brevet EP-A-1 321 130.

Au sens de la présente invention, le terme « (méth)acrylate » englobe aussi bien le terme acrylate que le terme méthacrylate. De même, le terme « acide (méth)acrylique » englobe aussi bien l'acide acrylique que l'acide méthacrylique.

Les monomères (a) utilisables pour la préparation du copolymère (méth)acrylique (i) utilisé dans la composition selon l'invention sont les monomères de (méth)acrylates de butyle tels que par exemple les (méth)acrylates de n-butyle, les (méth)acrylates de t-butyle, les (méth)acrylates d'isobutyle, les (méth)acrylates de 2-décylbutyle et les (méth)acrylates de 2-méthylbutyle.

De préférence, les monomères (a) sont choisis parmi l'acrylate de n-butyle.

Les monomères (a) représentent généralement de 15 à 50 % en poids, mieux de 30 à 40 % en poids, par rapport à la quantité totale de monomères utilisés.

Les monomères (b) utilisables pour la préparation du copolymère (méth)acrylique (i) utilisé dans la composition selon l'invention sont choisis parmi les monomères de (méth)acrylate d'hydroxy(C₁-C₄)alkyle, tels que par exemple les (méth)acrylates de 2-hydroxyéthyle, les (méth)acrylates d'hydroxypropyle, les (méth)acrylates de 2,3-dihydroxypropyle, les (méth)acrylates de 4-hydroxybutyle, et les (méth)acrylates de 2-hydroxypropyle.

De préférence, les monomères (b) sont choisis parmi les monomères d'acrylate de 2-hydroxyéthyle, de méthacrylate de 2-hydroxyéthyle et d'acrylate d'hydroxypropyle et plus particulièrement le méthacrylate de 2-hydroxyéthyle.

Les monomères (b) représentent généralement de 2 à 50 % en poids, de préférence de 2 à 25 % en poids, mieux de 2 à 10% en poids, par rapport à la quantité totale de monomères utilisés. Selon un autre mode de réalisation de mise en oeuvre distinct, les monomères (b) constituent de 10 à 50% en poids, de préférence de 10 à 30 % en poids, par rapport à la quantité totale de monomères utilisés.

Les monomères (c) éventuellement utilisables pour la préparation du copolymère (méth)acrylique (i) utilisé dans la composition selon l'invention peut être choisi parmi (i') les monomères de (méth)acrylate d'alkyle, de préférence les (méth)acrylates d'alkyle en C₁-C₂₄, (ii') les monomères polaires et (iii') les monomères à insaturation éthylénique polymérisables par voie radicalaire libre.

Les monomères de (méth)acrylate d'alkyle (i') peuvent inclure par exemple les (méth)acrylates de méthyle, les (méth)acrylates d'isobornyle, les (méth)acrylates d'éthyle, les (méth)acrylates d'iso-octyle, les (méth)acrylates de 2-éthylhexyle, les (méth)acrylates de lauryle, les (méth)acrylates d'octa-décyle, et des mélanges de ceux-ci. Dans un autre mode de mise en oeuvre, les motifs issus des monomères de (méth)acrylate de 2-éthylhexyle constituent de 30 à 80 % en poids de la quantité totale de monomères utilisés.

Les monomères polaires (ii') utiles incluent l'acide (méth)acrylique, l'acide itaconique, la N-vinylpyrrolidone, le N-vinylcaprolactame, les (méth)acrylamides substitués (tels que les N,N-diméthyl(méth)acrylamides et le N-octyl(méth)acrylamide), le (méth)acrylate de diméthylaminoéthyle, le (méth)acrylonitrile, le (méth)acrylate de 2-carboxyéthyle, l'anhydride maléique, et des mélanges de ceux-ci. Un autre exemple de monomères polaires (ii') utiles est le monoacrylate de méthoxypolyéthylèneglycol 550 disponible auprès de Sartomer Co. sous le nom commercial CD553.

Les monomères utiles à insaturation éthylénique polymérisables par voie radicalaire libre (iii') incluent le styrène et des esters de vinyle en C₁-C₄ tels que l'acétate de vinyle, le propionate de vinyle, et des mélanges de ceux-ci. Dans encore un autre mode de mise en oeuvre, les motifs issus de monomères à insaturation éthylénique polymérisable par voie radicalaire copolymérisable (iii') sont présents dans une concentration allant jusqu'à 30 % en poids par rapport au poids total de tous les monomères.

De préférence, les monomères (c) éventuellement utilisables pour la préparation du copolymère (méth)acrylique (i) utilisé dans la composition selon l'invention est le (méth)acrylate de 2-éthylhexyle.

Les monomères (c) éventuellement utilisables pour la préparation du copolymère (méth)acrylique (i) utilisé dans la composition selon l'invention constituent par exemple jusqu'à 80 % en poids, de préférence de 30 à 80 % en poids, mieux encore de 50 à 70 % en poids, par rapport à la quantité totale de monomères utilisés.

Selon un autre mode de réalisation, les monomères (c) peuvent constituer jusqu'à 50 % en poids, de préférence jusqu'à 30 % en poids, par rapport à la quantité totale de monomères utilisés.

De préférence, le ou les copolymères (méth)acryliques (i) comprennent uniquement les monomères a), b) et c).

De préférence, le ou les copolymères (méth)acryliques (i) sont non siliconés, c'est-à-dire qu'ils ne comprennent pas dans leur structure d'atome de silicium.

Le ou les copolymères (méth)acryliques (i) utilisés dans la composition selon l'invention sont préparés à partir d'un mélange de monomères pouvant contenir un ou plusieurs agents de réticulation polyfonctionnels.

Au sens de la présente invention, on entend par « agent de réticulation polyfonctionnel », un agent de réticulation ayant une fonctionnalité moyenne supérieure à 1, par exemple supérieure à 1,8 et de préférence supérieure à 2,0. Cependant, la fonctionnalité moyenne est inférieure à 6, par exemple inférieure à 4, et de préférence égale ou inférieure à 3.

Des exemples d'agents de réticulation incluent, mais sans s'y limiter, ceux choisis parmi le divinylbenzène, les diacrylates d'alkyle (tels que ceux choisis parmi le diacrylate de 1,2-éthylèneglycol, le diacrylate de 1,4-butanediol, le diacrylate de 1,6-hexanediol, le diacrylate de 1,8-octanediol et le diacrylate de 1,12-dodécanediol), les triacrylates d'alkyle et les tétra-acrylates d'alkyle (tels que le triacrylate de triméthylolpropane et le tétra-acrylate de pentaérythritol), les cétones aromatiques à insaturation monoéthylénique (telles que la 4-acryloxybenzophénone), les aziridine-amides multifonctionnels (tels que la 1,1'-(1,3-phénylènedicarbonyl)bis[2-méthylaziridine], la 2,2,4-triméthyladipoylbis[2-éthylaziridine], la 1,1'-azélaoylbis[2-méthylaziridine] et la 2,4,6-tris(2-éthyl-1-aziridinyl)-1,3,5-triazine), les réticulants à ions métalliques (tels que le cuivre, le zinc, le zirconium et le chrome) et des mélanges de ceux-ci. Dans un mode de mise en oeuvre, les réticulants à ions métalliques sont choisis parmi les esters chélatés d'acide ortho-titanique vendus sous la désignation commerciale TYZOR et disponibles sur le marché auprès de E.I. du Pont de Nemours Co. Dans un autre mode de mise en oeuvre, le TYZOR est le TYZOR AA, qui est l'acétylacétonate de titane.

De préférence, l'agent de réticulation est le diacrylate de 1,6-hexanediol.

Les agents de réticulation, lorsqu'ils sont utilisés, peuvent représenter jusqu'à 10 parties en poids, typiquement de 0,1 à 2 parties en poids du mélange copolymérisable total sur la base de 100 parties en poids des monomères cités dans (a), (b) et (c), lorsqu'ils sont présents.

Le ou les copolymères (méth)acryliques (i) utilisés dans la composition selon l'invention peuvent être éventuellement obtenus avec un ou plusieurs amorceurs hydrosolubles et/ou oléo-solubles, qui sont utilisés pour obtenir des copolymères (méth)acryliques sous forme d'émulsions.

De tels amorceurs, par exposition à la chaleur, génèrent des radicaux libres qui amorcent la (co)polymérisation des monomères de (méth)acrylate de butyle, des monomères de (méth)acrylate d'hydroxyalkyle et les composants éventuels de comonomère et d'agent de réticulation.

Selon un mode de mise en oeuvre, on utilise un ou plusieurs amorceurs hydrosolubles. Par « amorceur hydrosoluble », on entend au sens de la présente invention, des amorceurs ayant une solubilité dans l'eau mesurée à 25°C au moins égale à 0,1 gramme/litre (g/L) (obtention d'une solution macroscopiquement isotrope et transparente, colorée ou non). Cette solubilité est de préférence supérieure ou égale à 1 g/L.

Les amorceurs hydrosolubles convenables incluent, mais sans s'y limiter, ceux choisis parmi le persulfate de potassium, le persulfate d'ammonium, le persulfate de sodium, et des mélanges de ceux-ci ; les amorceurs oxydo-réducteurs tels que le produit de réaction des persulfates cités ci-dessus et des agents réducteurs tels que ceux choisis parmi le métabisulfite de sodium et le bisulfite de sodium ; et l'acide 4,4'-azobis(4-cyanopentanoïque) et ses sels solubles (par ex. de sodium, de potassium). Dans un autre mode de mise en oeuvre, l'amorceur hydrosoluble est le persulfate de potassium.

De manière alternative, les amorceurs oléo-solubles convenables incluent, mais sans s'y limiter, ceux choisis parmi les composés azoïques tels que VAZO 64 (le 2,2'-azobis(isobutyronitrile) et VAZO 52 (le 2,2'-azobis(2,4-diméthylpentanenitrile)), tous deux disponibles auprès de E.I. du Pont de Numours Co. ; et les peroxydes tels que le peroxyde de benzoyle, le peroxyde de lauroyle et des mélanges de ceux-ci. Dans un mode de mise en oeuvre, l'amorceur thermique oléo-soluble est le 2,2'-azobis(isobutyronitrile).

Lorsqu'il(s) est (sont) utilisé(s), l'(les) amorceur(s) peut (peuvent) représenter de 0,05 à 1 partie en poids, également de 0,1 à 0,5 partie en poids sur la base de 100 parties en poids du mélange copolymérisable total.

Dans un autre mode de mise en oeuvre, le ou les copolymères (méth)acryliques (i) utilisés dans la composition selon l'invention peuvent être éventuellement obtenus avec un ou plusieurs agents de transfert de chaîne.

Des exemples d'agents de transfert de chaîne utiles incluent, mais sans s'y limiter, ceux choisis parmi le tétrabromure de carbone, les alcools, les mercaptans, et des mélanges de ceux-ci.

De manière particulièrement préférée, l'agent de transfert de chaîne est choisi parmi l'iso-octylthioglycolate et le tétrabromure de carbone.

Le mélange copolymérisable peut en outre comprendre jusqu'à 0,5 partie en poids d'un ou plusieurs agents de transfert de chaîne, typiquement de 0,01 % en poids à 0,5 partie en poids, s'ils sont utilisés, également de 0,05 partie en poids à 0,2 partie en poids, sur la base de 100 parties en poids du mélange copolymérisable total.

Plus préférentiellement, le ou les copolymères (méth)acryliques (i) utilisés dans la composition selon l'invention comprennent :
- (a) de 30 à 40 % en poids de motifs issus d'un ou plusieurs monomères choisis parmi les monomères d'acrylate de n-butyle,
- (b) de 2 à 10 % en poids de motifs issus d'un ou plusieurs monomères choisis parmi les monomères de méthacrylate de 2-hydroxyéthyle, et
- (c) de 50 à 70 % en poids de motifs issus d'un monomère d'acrylate de 2-éthylhexyle.

De manière encore plus préférée, le copolymère (méth)acrylique (i) utilisé dans la composition selon l'invention comprend :
- (a) 35 % en poids de motifs issus d'un monomère d'acrylate de butyle,
- (b) 5 % en poids de motifs issus d'un monomère de méthacrylate de 2-hydroxyéthyle,
- (c) 60 % en poids de motifs issus d'un monomère de d'acrylate de 2-éthylhexyle.

Un tel composé est commercialisé par la société 3M sous la dénomination CPC-00960 ACP 45 Acrylates Copolymer.

Les pourcentages en poids des motifs (a), (b) et (c) sont basés sur le poids total de chaque type de monomère utilisé par rapport au poids total de tous les monomères utilisés.

Le ou les copolymères (méth)acryliques (i) peuvent être présents en une quantité allant de 0,01 à 40% en poids, de préférence allant de 0,1 à 15 % en poids, et plus préférentiellement allant de 0,1 à 2% en poids, du poids total de la composition pour donner un effet repositionnable.

Le ou les copolymères (méth)acryliques (i) peuvent être sous forme d'émulsion ou de dispersion. Toutes les émulsions comprennent une phase continue et au moins une phase dispersée.

Par « dispersion », on entend généralement un système multiphasique où au moins une phase contient des particules discrètes distribuées dans une phase externe liquide, solide ou gazeuse. Une partie du polymère peut exister sous forme de particules discrètes dans une phase aqueuse. Les dispersions sont possibles grâce à l'utilisation de certains composants qui sont insolubles dans le système aqueux. Dans une dispersion, ce n'est pas nécessairement l'ensemble du polymère qui doit être insoluble dans l'eau ; une partie du polymère peut être soluble dans le mélange aqueux. Il peut être souhaitable qu'une dispersion reste stable dans des conditions ambiantes.

Dans un mode de mise en oeuvre, les dispersions sont stables à température ambiante pendant plus d'environ 30 jours, par exemple pendant plus d'environ 90 jours, de préférence pendant plus d'environ 180 jours, et mieux encore pendant plus d'environ 360 jours. Une dispersion est jugée stable dès lors que les particules discrètes de la phase interne restent distribuées dans l'ensemble de la substance de masse (phase externe).

Dans un mode de mise en oeuvre, de telles dispersions peuvent être mélangées avec d'autres dispersions ou d'autres additifs connus tels que des plastifiants, des pigments (tels que le noir de carbone), des sols de silice et d'autres agents de nivellement, agents mouillants, agents anti-mousse, et stabilisants connus.

Le ou les copolymères (méth)acryliques (i) utilisés dans la composition selon l'invention peuvent être préparés par des techniques de polymérisation conventionnelles, telles que la polymérisation en émulsion, comme cela est bien connu dans le domaine des polymères.

La polymérisation par l'intermédiaire des techniques d'émulsion peut nécessiter la présence d'un ou plusieurs émulsifiants afin de faciliter l'émulsification du mélange de monomères (que l'on peut également appeler agents émulsifiants ou tensioactifs). Les émulsifiants utiles pour la présente invention incluent ceux choisis parmi les tensioactifs anioniques, les tensioactifs non ioniques, et des mélanges de ceux-ci.

Les tensioactifs anioniques utiles comme émulsifiants incluent, mais sans s'y limiter, ceux dont la structure moléculaire inclut au moins une moitié hydrophobe choisie parmi les alkyles en C₆ à C₁₂, les alkylaryles en C₆ à C₁₂, et les alcényles en C₆ à C₁₂ et au moins un groupement anionique choisi parmi les sulfates, les sulfonates, les phosphates, les polyoxyéthylènesulfates, les polyoxyéthylènesulfonates, les polyoxyéthylènephosphates, etc., et les sels de tels groupements. Dans un mode de mise en oeuvre, lesdits sels sont choisis parmi les sels de métaux alcalins, les sels d'ammonium, les sels d'amino tertiaire, etc. Des exemples commerciaux représentatifs des tensioactifs anioniques utiles comme émulsifiants incluent les laurylsulfates de sodium, disponibles auprès de Stepan Chemical Co. sous forme de POLYSTEP B-3 ; les lauryléthersulfates de sodium, disponibles auprès de Stepan Chemical Co. sous forme de POLYSTEP B-12 ; les dodécylbenzènesulfonates de sodium, disponibles auprès de Rhodia Chimie sous forme de SIPONATE DS-10 ; et les alkylènepolyalcoxyammonium sulfates, disponibles auprès de PPG Industries sous forme de MAZON SAM-211.

Les tensioactifs non ioniques utiles comme émulsifiants incluent, mais sans s'y limiter, ceux dont la structure moléculaire comprend un produit de condensation d'une moitié aliphatique organique et/ou alkyle aromatique avec un oxyde d'alkylène hydrophile tel que l'oxyde d'éthylène. Le coefficient HLB (balance lipophile-hydrophile) de tensioactifs non ioniques utiles comme émulsifiants est d'environ 10 ou supérieur, par exemple d'environ 10 à environ 20. Le coefficient HLB d'un tensioactif est une expression de la balance de la taille et de la résistance des groupements hydrophiles (hydro-attracteurs ou polaires) et des groupements lipophiles (oléo-attracteurs ou non polaires) du tensioactif. Des exemples commerciaux des tensioactifs non ioniques utiles dans la présente invention incluent, mais sans s'y limiter, les nonylphénoxy ou octylphénoxy poly(éthylèneoxy)éthanols disponibles auprès de Rhodia Chimie sous forme respectivement de la série IGEPAL CA ou CO ; les éthoxylates d'alcools secondaires en C₁₁-C₁₅ disponibles auprès d'Union Carbide sous forme de la série TERGITOL 15-S ; et les esters d'acides gras du sorbitane oxyéthylénés disponibles auprès d'ICI Chemicals sous forme de la série de tensioactifs TWEEN.

Dans un mode de mise en oeuvre, une polymérisation en émulsion selon l'invention est réalisée en présence d'un ou plusieurs tensioactifs anioniques comme émulsifiants. Une gamme utile de concentration d'émulsifiant est de 0,5 à 8 % en poids, par exemple de 1 à 5 % en poids, sur la base du poids total de tous les monomères.

Selon un mode de réalisation de l'invention, les copolymères (méth)acryliques (i) sont des émulsions ou des dispersions de copolymères (méth)acryliques.

Les émulsions et les dispersions de copolymères (méth)acryliques (i) peuvent être préparées par un procédé semi-continu de polymérisation en émulsion. Dans le procédé, une fiole est chargée d'un mélange de monomères amorce comprenant de l'eau désionisée (DI), du tensioactif, des monomères de (méth)acrylate de butyle (a), des monomères de (méth)acrylate d'hydroxyalkyle (b), et les composants éventuels tels que les monomères copolymérisables (c), des agents de réticulation polyfonctionnels, des agents de transfert de chaîne, des modificateurs de pH et d'autres additifs. Le mélange est agité et chauffé sous une atmosphère inerte telle qu'une couverture d'azote. Lorsque le mélange a atteint la température d'induction, typiquement d'environ 50°C à environ 70°C, le premier amorceur est ajouté pour amorcer la polymérisation et on laisse la réaction se dérouler de façon exothermique. Lorsque la réaction d'amorçage est terminée, la température du lot (batch) est ensuite augmentée jusqu'à la température de réaction d'alimentation, d'environ 70°C à environ 85°C. A la température de réaction d'alimentation, la pré-émulsion de monomères comprenant de l'eau DI, du tensioactif, des monomères de (méth)acrylate de butyle (a), des monomères de (méth)acrylate d'hydroxyalkyle (b), et des composants éventuels tels que les monomères copolymérisables (c), des agents de réticulation polyfonctionnels, des agents de transfert de chaîne, et d'autres additifs est ajoutée à la fiole agitée sur une période de temps, typiquement de 2 à 4 heures, pendant que la température est maintenue. A la fin de la réaction d'alimentation, la deuxième charge d'amorceur, si on l'utilise, est ajoutée à la réaction pour réduire encore les monomères résiduels dans l'émulsion/la dispersion. Après une heure supplémentaire de chauffage, le mélange est refroidi jusqu'à température ambiante (environ 23°C) et l'émulsion/la dispersion est récupérée pour une évaluation.

Dans un mode de mise en oeuvre, le pH de l'émulsion/la dispersion est d'environ 2 à environ 3. L'acidité de l'émulsion/la dispersion peut être modifiée suivant la formation de l'émulsion/la dispersion à l'aide d'un ou plusieurs modificateurs de pH tels que des solutions basiques (par ex. des solutions d'hydroxyde de sodium, d'hydroxyde d'ammonium, etc.) ou des solutions tampons (par ex. du bicarbonate de sodium, etc.) à des taux d'acidité inférieurs. Dans un autre mode de mise en oeuvre, le pH est de 7 ou inférieur. Dans encore un autre mode de mise en oeuvre, le pH est dans la gamme de 2 à 6.

Dans un mode de mise en oeuvre de l'invention, les copolymères (méth)acryliques (i) peuvent être neutralisés dans l'émulsion/la dispersion et/ou dans la composition par un ou plusieurs agents neutralisants. Les agents neutralisants convenables peuvent être choisis parmi des bases organiques, minérales ou organo-minérales, telles que les aminométhylpropanols, les hydroxydes de sodium et de potassium, les amines primaires, secondaires et tertiaires, les ammoniacs, les dérivés de ceux-ci, et les associations de ceux-ci.

Selon un mode de réalisation, les émulsions (méth)acryliques de l'invention peuvent également contenir un ou plusieurs additifs classiques, tels que des plastifiants, des colorants, des anti-oxydants, et des stabilisants des U.V. De tels additifs peuvent être utilisés s'ils n'altèrent pas les propriétés repositionnables de la composition.

Selon un mode de mise en oeuvre de l'invention, le copolymère (méth)acrylique (i) a une température de transition vitreuse (Tg) allant d'environ - 100°C à environ 15°C. Selon la présente invention, la Tg du copolymère (méth)acrylique est obtenue à la suite de l'application du copolymère (méth)acrylique (i) dans un milieu aqueux ou hydro-alcoolique sur une matrice, puis du séchage jusqu'à poids constant. La température de transition vitreuse est déterminée par la méthode d'analyse thermique différentielle (DSC).

Dans un mode de mise en oeuvre de l'invention, le copolymère (méth)acrylique (i) est choisi parmi les copolymères (méth)acryliques non ioniques et faiblement anioniques. Faiblement anionique signifie que le taux de motifs issus de monomères anioniques dans le copolymère est inférieur à 5 % en poids.

Dans tout ce qui suit, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

Les silicones utilisées dans la composition selon l'invention peuvent être volatiles ou non, solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les organopolysiloxanes sont définis plus en détail dans l'ouvrage de Walter NOLL "Chemistry and Technology of Silicones" (1968) Academic Press. Ils peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.

On peut également citer les cyclocopolymères du type diméthylsiloxane/méthylalkylsiloxane, tel que la "SILICONE VOLATILE FZ 3109" commercialisée par la société UNION CARBIDE, de structure chimique :

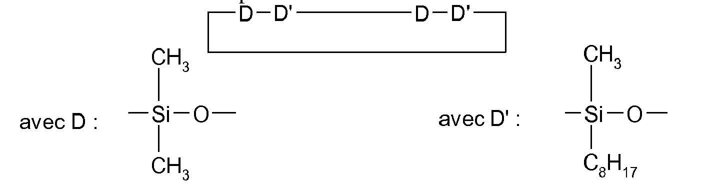

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Par silicone non volatile, on entend au sens de la présente invention, toute silicone dont le nombre d'atomes de silicium est supérieure à 7.

Parmi les silicones non volatiles, on peut notamment citer des polyalkylsiloxanes, des polyarylsiloxanes, des polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société Rhodia ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société Rhodia.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyl-diphénylsiloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHODIA,
- les huiles des séries RHODORSIL 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes de polydiméthylsiloxane,
- les gommes de polydiméthylsiloxanes/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane/diphénylsiloxane/méthylvinylsiloxane.

Peuvent également être employés des mélanges de silicones tels que :
- les mélanges formés à partir d'une gomme de polydiméthylsiloxane hydroxylée en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux
dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC Fluid 190 ou DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

De préférence, la silicone est une silicone aminée.

Par silicone aminé, on entend au sens de la présente invention, toute silicone comportant au moins une fonction amine primaire, secondaire, tertiaire ou un groupement ammonium quaternaire.

Les silicones aminées utilisées dans la composition sous forme de film selon la présente invention sont choisies parmi :

### (a) les composés répondant à la formule (I) suivante :

(R¹)ₐ(T)₃₋ₐ-Si[OSi(T)₂]ₙ-[OSi(T)_{b}(R¹)₂-b]ₘ-OSi(T)₃₋ₐ-(R¹)ₐ (I)

dans laquelle,
T est un atome d'hydrogène, ou un radical phényle, hydroxyle (-OH), ou alkyle en C₁-C₈, et de préférence méthyle ou alcoxy en C₁-C₈, de préférence méthoxy,
a désigne le nombre 0 ou un nombre entier de 1 à 3, et de préférence 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R₁ est un radical monovalent de formule -C_{q}H_{2q}L dans laquelle q est un nombre de 2 à 8 et L est un groupement aminé éventuellement quaternisé choisi parmi les groupements :
-N(R²)-CH₂-CH₂-N(R²)₂ ;
-N(R²)₂ ; -N⁺(R²)₃ Q- ;
-N⁺(R²) (H)₂ Q- ;
-N⁺(R²)₂HQ- ;
-N(R²)-CH₂-CH₂-N⁺(R²)(H)₂ Q-,
   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

En particulier, les silicones aminées correspondant à la définition de la formule (I) sont choisies parmi les composés correspondant à la formule suivante (II) : 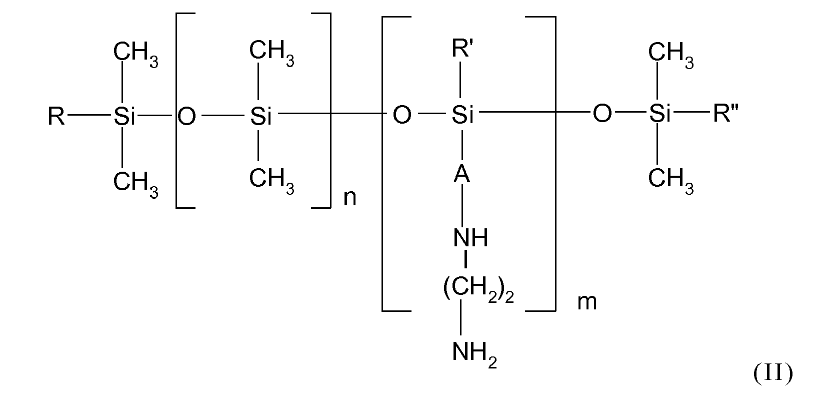 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy /alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (I) est en particulier le polymère dénommé dans le dictionnaire CTFA "triméthylsilylamodiméthicone", répondant à la formule (III) suivante: 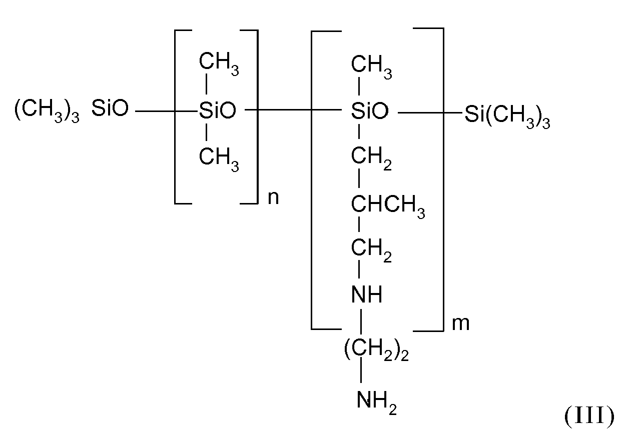 dans laquelle n et m ont les significations données ci-dessus conformément à la formule (II).

De tels composés sont décrits par exemple dans EP 95238; un composé de formule (I) est par exemple vendu sous la dénomination Q2-8220 par la société OSI.

### (b) les composés répondant à la formule (IV) suivante :

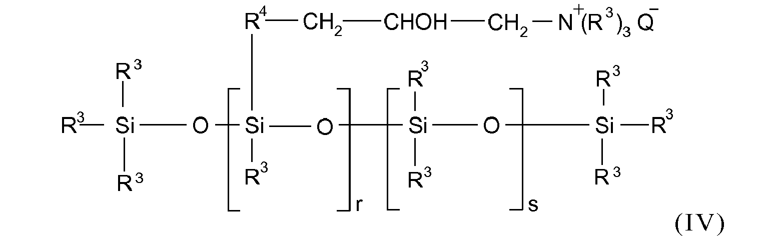

dans laquelle,
R³ représente un radical hydrocarboné monovalent en C₁-C₁₈, et en particulier un radical alkyle en C₁-C₁₈, ou alcényle en C₂-C₁₈, par exemple méthyle ;
R⁴ représente un radical hydrocarboné divalent, notamment un radical alkylène en C₁-C₁₈ ou un radical alkylèneoxy divalent en C₁-C₁₈, par exemple en C₁-C₈;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels composés sont décrits plus particulièrement dans le brevet US 4185087.

Un composé entrant dans cette classe est celui vendu par la Société Union Carbide sous la dénomination "Ucar Silicone ALE 56".

### c) les silicones ammonium quaternaire de formule (V) :

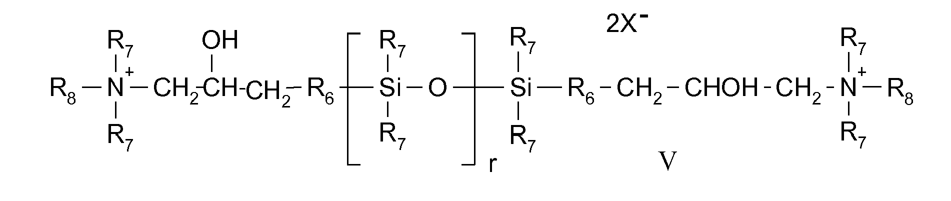

dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇ ;
X- est un anion tel qu'un ion halogénure, notamment chlorure ou un sel d'acide organique (acétate ...);
r représente une valeur statistique moyenne de 2 à 200 et en particulier de 5 à 100 ;

Ces silicones sont par exemple décrites dans la demande EP-A-0530974.

Selon l'invention, on peut également utiliser des polymères du type silicone greffée comprenant une portion polysiloxane et une portion comprenant une chaîne organique non siliconée, l'une des deux portions constituant la chaîne principale du polymère et l'autre étant greffée sur la chaîne principale. Ces polymères sont divulgués, par exemple, dans les documents EP A 0 412 704, EP-A-0 412 707, EP-A-0 640 105, WO 95/00578, EP-A-0 582 152 et WO 93/23009 et les brevets U.S. n° 4 693 935, 4 728 571 et 4 972 037. Ces polymères sont par exemple anioniques ou non ioniques.

De tels polymères sont par exemple des copolymères susceptibles d'être obtenus par polymérisation radicalaire à partir du mélange de monomères comprenant :
a) environ 50 % à environ 90 % en poids d'acrylate de tert-butyle ;
b) 0 % à environ 40 % en poids d'acide acrylique ;
c) environ 5 % à environ 40 % en poids de macromère siliconé de formule : 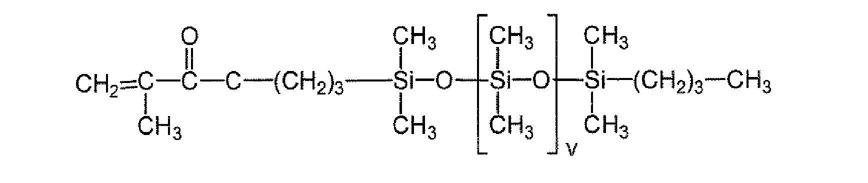
dans laquelle v est un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'une chaîne de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'une chaîne de raccordement de type thiopropylène, des motifs polymères du type poly(méth)acrylate d'isobutyle.

La ou les silicones (ii) utilisées dans la composition selon l'invention peuvent être présentes dans une quantité variant de 0,01 à 20%, de préférence en une quantité variant de 0,1 à 10 % et encore plus préférentiellement dans une quantité variant de 0,5 à 5 % en poids, par rapport au poids total de la composition.

De préférence, la composition de coiffage selon l'invention est une composition rincée. On entend par « composition rincée » toute composition qui est formulée pour être rincée immédiatement ou après un temps de pose inférieur à 30 minutes, de préférence inférieur à 10 minutes, après application sur les cheveux.

La composition rincée peut se présenter sous toutes les formes classiques de compositions cosmétiques rincées y compris, mais sans s'y limiter, les shampoings, après-shamppoings, lotions de rinçage des cheveux, compositions de permanente, compositions de teinture des cheveux, produits à utiliser avant ou après un traitement de teinture des cheveux, produits à utiliser avant ou après un traitement de permanente, compositions de défrisage, produits à utiliser avant ou après un traitement de défrisage, et des associations de ceux-ci. Un shampoing présente un effet nettoyant sur les cheveux et peut également présenter un effet conditionneur. Un après-shampooing présente un effet conditionneur sur les cheveux sans effet nettoyant marqué.

De préférence, la composition selon l'invention est une composition rincée, en particulier un shampooing ou un après-shampooing.

La composition peut comprendre tout véhicule cosmétiquement acceptable qui ne gêne pas substantiellement les propriétés de la composition.

Par véhicule cosmétiquement acceptable, on entend au sens de la présente invention, un véhicule compatible avec les fibres kératiniques et notamment les cheveux.

Le choix de véhicule est adapté au procédé d'application choisi. Dans un mode de mise en oeuvre, le véhicule cosmétiquement acceptable peut être choisi parmi l'eau, les solvants miscibles à l'eau tels que les alcools inférieurs, par ex. les alcools aliphatiques à chaîne ramifiée et droite en C₁ à C₄, et des associations de ceux-ci. Dans un mode de mise en oeuvre, le copolymère (méth)acrylique (i) est insoluble dans le véhicule cosmétiquement acceptable.

Le véhicule peut également comprendre un ou plusieurs solvants supplémentaires. Par exemple, on peut utiliser d'autres solvants s'évaporant rapidement, tels que l'hexaméthyldisiloxane (HMDS) ; les silicones cycliques (D4 et D5) ; les alcanes en C₄ - C₁₀ dont les isoparaffines telles que Permethyl 97A et Isopar C ; l'acétone ; les hydrofluoroéthers (HFE), etc.

La composition selon l'invention peut en outre comprendre un ou plusieurs constituants connus en cosmétique qui ne gênent pas substantiellement les propriétés de la composition. De tels constituants peuvent être choisis parmi les, mais ne sont pas limités aux : polymères anioniques, agents réducteurs (tels que les thiols) ; silanes (tels que l'aminopropyltriéthoxysilane) ; matières grasses ; épaississants ; plastifiants ; agents hydratants ; filtres solaires (tels que filtres UV) ; agents actifs de soin des cheveux ; parfums ; conservateurs ; polyols ; protéines ; provitamines ; vitamines ; colorants ; et agents de modification du pH.

Les compositions peuvent également contenir un ou plusieurs agents conditionneurs autres que les silicones (ii). On entend par « agent conditionneur » tel qu'utilisé ici tout agent dont la fonction est d'améliorer les propriétés cosmétiques des cheveux, par exemple la douceur, la facilité de démêlage, le toucher, et l'absence d'électricité statique. Dans une variante, l'agent conditionneur est choisi parmi les polymères cationiques, amphotères ou non ioniques.

Selon un mode de réalisation préférentiel, ledit agent conditionneur est choisi parmi les polymères tels que les polymères cationiques, amphotères (tels que zwitterioniques), et non ioniques, de préférence les polymères cationiques et des associations de ceux-ci. On entend par « polymère » tel qu'utilisé ici les homopolymères et les copolymères, les copolymères étant issus de plus d'un type de monomère, par exemple de deux, trois, quatre types de monomères différents ou plus.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (VI), (VII), (VIII) ou (IX) suivantes: 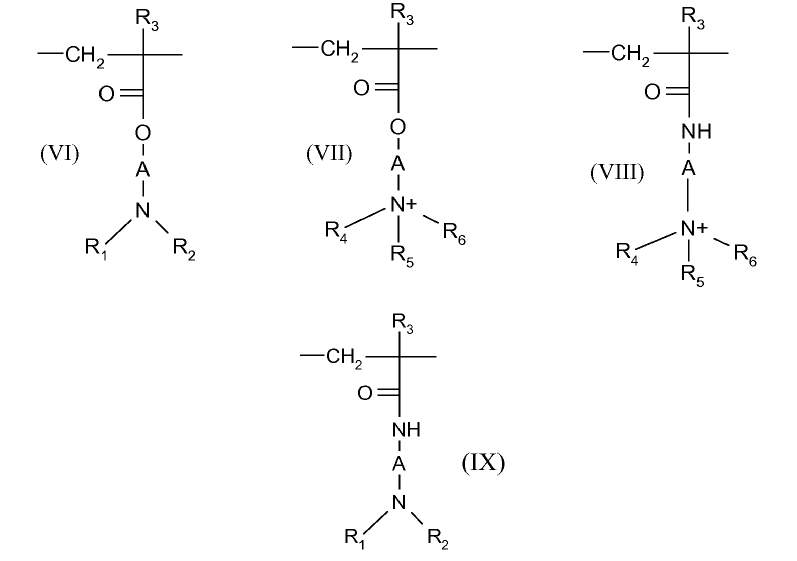 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un halogénure de diméthyle;
   - les copolymères d'acrylamide et de chlorure de méthacryloyloxyéthyltriméthylammonium décrits par exemple dans la demande de brevet EP-A-080976;
   - le copolymère d'acrylamide et de méthosulfate de méthacryloyloxyéthyltriméthylammonium;
   - les copolymères vinylpyrrolidone / acrylate ou méthacrylate de dialkylaminoalkyle quaternisés ou non. Ces polymères sont décrits en détail dans les brevets français 2.077.143 et 2.393.573;
   - les terpolymères méthacrylate de diméthyl amino éthyle/vinylcaprolactame/ vinylpyrrolidone;
   - les copolymère vinylpyrrolidone / méthacrylamidopropyl diméthylamine;
   - les copolymères vinylpyrrolidone / méthacrylamide de diméthylaminopropyle quaternisés
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société CIBA.
(2) Les polysaccharides cationiques et en particulier ceux choisis parmi :
   (a) les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
   (b) les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylmidopropyl triméthylammonium, de diméthyl-diallylammonium.
   (c) les polygalactomannanes cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(4) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(7) Les cyclopolymères d'alkyl diallyl amine ou de dialkyl diallyl ammonium tels que les homopolymères ou copolymères comportant comme constituant principal de la chaîne des motifs répondant aux formules (X) ou (XI) : 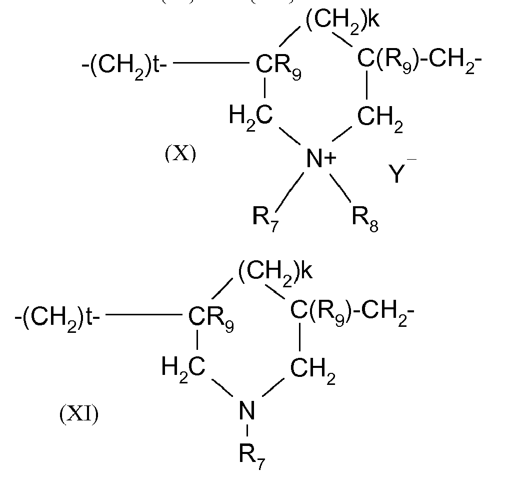 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
(8) Le polymère de diammonium quaternaire contenant des motifs récurrents répondant à la formule : 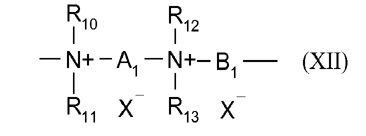 formule (XII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
   a) un reste de glycol de formule : -O-Z-O-, où Z désigne un radical hydrocarboné linéaire ou ramifié ou un groupement répondant à l'une des formules suivantes :
      - (CH₂-CH₂-O)x-CH₂-CH₂-
      - [CH₂-CH(CH₃)-O]y-CH₂-CH(CH₃)-
      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-,
      où Y désigne un radical hydrocarboné linéaire ou ramifié, ou bien le radical bivalent
      -CH₂-CH₂-S-S-CH₂-CH₂- ;
   d) un groupement uréylène de formule : -NH-CO-NH- .

De préférence, X⁻ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (XIII) suivante: 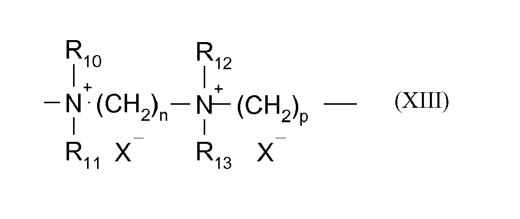 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(9) Les polymères de poly(ammonium quaternaire) constitués de motifs récurrents de formule (XIV) : 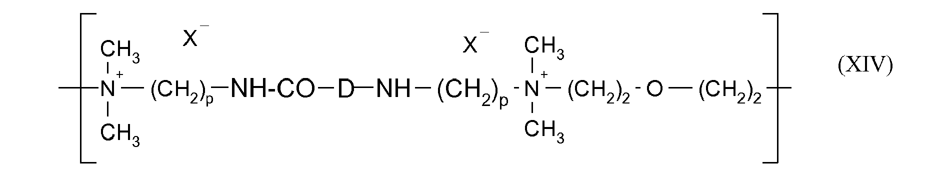 dans laquelle p désigne un nombre entier variant de 1 à 6 environ, D peut être nul ou peut représenter un groupement -(CH₂)ᵣ - CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
(10) Les polymères quaternaires de vinylpyrrolidone et de vinylimidazole.
(11) Les polyamines comme le produit référencé sous le nom de " POLYETHYLENEGLYCOL (15) TALLOW POLYAMINE " dans le dictionnaire CTFA.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques utilisables selon la présente invention, on préfère notamment :
- les dérivés d'éther de cellulose quaternaires tels que les produits commercialisés sous la dénomination « JR 400 » par la société Rhodia Chimie, les cyclopolymères, en particulier les homopolymères ou copolymères de chlorure de diméthyldiallylammonium, les homopolymères de sel de diallyldiméthylammonium et les copolymères de sel de diallyldiméthylammonium et d'acrylamide en particulier les chlorures, commercialisés sous les dénominations « MERQUAT 100 » « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société AMERCHOL, les homopolymères et les copolymères éventuellement réticulés de sel de (méth)acryloyloxyéthyltriméthylammonium, vendus par la société CIBA en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

Selon un mode de réalisation particulièrement préféré, les compositions selon l'invention comprennent un ou plusieurs polymères cationiques choisis parmi les homopolymères ou les copolymères du chlorure de diméthyldiallylammonium.

Selon l'invention, le(s) polymère(s) cationique(s) peut (peuvent) représenter de 0,001 % à 20 % en poids, par exemple de 0,01 % à 10 % en poids, encore par exemple de 0,1 % à 3 % en poids, par rapport au poids total de la composition.

Les polymères amphotères utilisables dans les compositions de la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl triméthyl ammonium.
   Le composé vinylique peut être également un sel de dialkyldiallylammonium tel que le chlorure de diméthyldiallylammonium.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
(3) Les polyaminoamides réticulés et alcoylés partiellement ou totalement dérivant de polyaminoamides de formule générale : 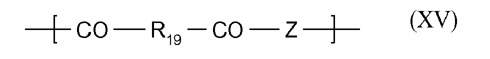 dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 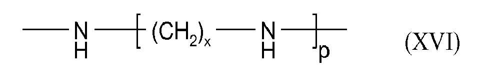 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XVI) ci-dessus, dans lequel x=2 et p=1 et qui dérive de l'éthylènediamine, ou le radical dérivant de la pipérazine : 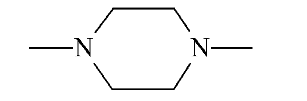
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 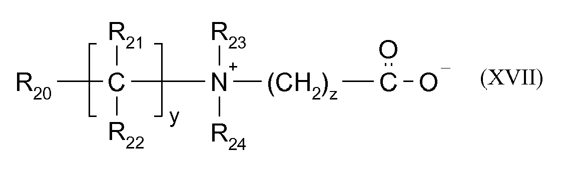 dans laquelle R₂₀ désigne un groupement insaturé polymérisable tel qu'un groupement acrylate, méthacrylate, acrylamide
   ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane comportant des motifs monomères répondant aux formules (XVIII), (XIX), (XX) suivantes : 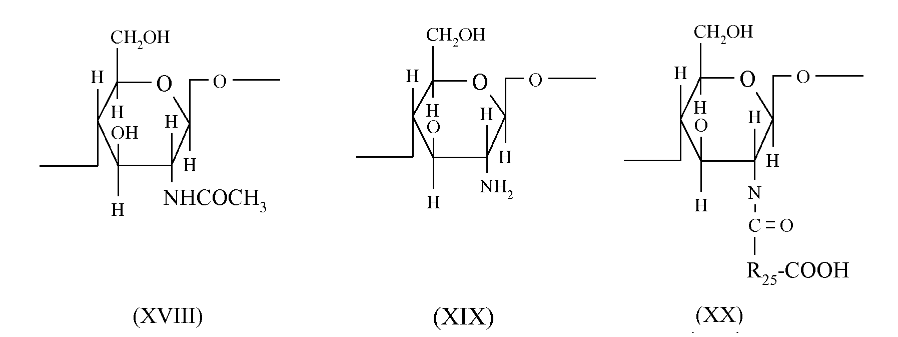 le motif (XVIII) étant présent dans des proportions comprises entre 0 et 30%, le motif (XIX) dans des proportions comprises entre 5 et 50% et le motif (XX) dans des proportions comprises entre 30 et 90%, étant entendu que dans ce motif (XX), R₂₅ représente un radical de formule : 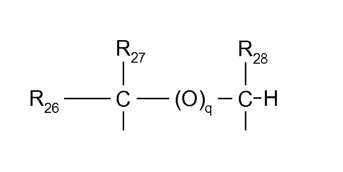 dans laquelle q désigne zéro ou 1 ;
   si q₌0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane.
(7) Les polymères répondant à la formule générale (XXI) tels que ceux décrits par exemple dans le brevet français 1 400 366 et comprenant des unités : 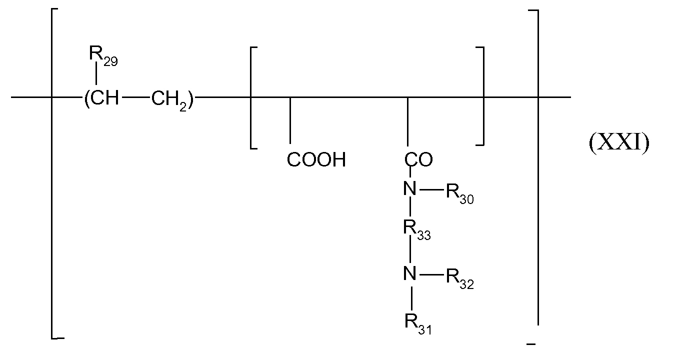 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂- , -CH₂-CH(CH₃)- , R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XXII)

      où D désigne un radical 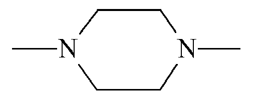 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XXIII)
   où D désigne un radical 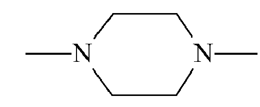 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un
   ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine
ou par semiestérification avec une N,N-dialcanolamine. Ces copolymères peuvent également comporter d'autres comonomères vinyliques tels que le vinylcaprolactame.

Parmi tous les polymères amphotères utilisables dans la composition selon la présente invention, on préfère notamment :
- le copolymère chlorure de diméthyldiallylammonium/acide acrylique (80/20) commercialisé sous la dénomination MERQUAT 280 DRY par la société CALGON (dénomination CTFA : POLYQUATERNIUM 22);
- le copolymère chlorure de diméthyldiallylammonium/acide acrylique (95/5) commercialisé sous la dénomination MERQUAT 295 DRY par la société CALGON (dénomination CTFA : POLYQUATERNIUM 22);
- le copolymère de chlorure de méthacrylamidopropyltrimonium, d'acide acrylique et d'acrylate d'éthyle, commercialisé sous la dénomination MERQUAT 2001 par la société CALGON (dénomination CTFA : POLYQUATERNIUM 47); et
- le terpolymère acrylamide/chlorure de diméthyldiallylammonium/acide acrylique, commercialisé sous la dénomination MERQUAT PLUS 3330 DRY par la société CALGON (dénomination CTFA : POLYQUATERNIUM 39).

Les polymères non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines vendues par la société Dow Chemical sous les noms PEOX 50 000, PEOX 200 000 et PEOX 500 000 ;
- les homopolymères d'acétate de vinyle tels que le produit vendu sous le nom Appretan EM par la société Hoechst ou le produit vendu sous le nom Rhodopas A 012 par la société Rhodia Chimie ;
- les copolymères d'acétate de vinyle et d'ester acrylique tels que le produit vendu sous le nom Rhodopas AD 310 par Rhodia Chimie;
- les copolymères d'acétate de vinyle et d'éthylène tels que le produit vendu sous le nom Appretan TV par la société Hoechst ;
- les copolymères d'acétate de vinyle et d'ester maléïque, par exemple de maléate de dibutyle tels que le produit vendu sous le nom Appretan MB Extra par la société Hoechst ;
- les copolymères de polyéthylène et d'anhydride maléïque ;
- les homopolymères d'acrylate d'alkyle et les homopolymères de méthacrylate d'alkyle tels que le produit vendu sous le nom Micropearl RQ 750 par la société Matsumoto ou le produit vendu sous le nom Luhydran A 848 S par la société BASF ;
- les copolymères d'esters acryliques tels que par exemple les copolymères de (méth)acrylates d'alkyle, tels que les produits vendus par la société Rohm & Haas sous les noms Primal AC-261 K et Eudragit NE 30 D, par la société BASF sous les noms Acronal 601, Luhydran LR 8833 ou 8845, et par la société Hoechst sous les noms Appretan N 9213 ou N 9212 ;
- les copolymères d'acrylonitrile et d'un monomère non ionique choisis par exemple parmi le butadiène et les (méth)acrylates d'alkyle ; on peut citer les produits vendus sous les noms Nipol LX 531 B par la société Nippon Zeon ou ceux vendus sous le nom CJ0601 B par la société Rohm & Haas ;
- les polyuréthanes tels que les produits vendus sous les noms Acrysol RM 1020 ou Acrysol RM 2020 par la société Rohm & Haas, et les produits Uraflex XP 401 UZ et Uraflex XP 402 UZ par la société DSM Resins ;
- les copolymères d'acrylate d'alkyle et d'uréthanne tels que le produit 8538-33 par la société National Starch ;
- les polyamides tels que le produit Estapor LO 11 vendu par la société Rhodia Chimie ;
- les gommes de guar non ioniques chimiquement modifiées ou non modifiées.

Les gommes de guar non ioniques non modifiées sont par exemple les produits vendus sous le nom Vidogum GH 175 par la société Unipectine et sous le nom Jaguar C par la société Meyhall.

Les gommes de guar non ioniques modifiées utilisables selon l'invention sont par exemple modifiées par des groupements hydroxyalkyle en C₁-C₆. On peut citer à titre d'exemples les groupements hydroxyméthyle, hydroxyéthyle, hydroxypropyle et hydroxybutyle.

Ces gommes de guar sont bien connues dans la technique antérieure et peuvent être préparées par exemple en faisant réagir des oxydes d'alcène correspondants tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les noms commerciaux Jaguar HP8, Jaguar HP60, Jaguar HP120, Jaguar DC 293, et Jaguar HP 105 par la société Meyhall et sous le nom Galactosol 4H4FD2 par la société Aqualon.

Les groupements alkyles des polymères non ioniques comprennent de 1 à 6 atomes de carbone, sauf mention contraire.

Il est également possible d'utiliser, comme polymères, des polyuréthanes fonctionnalisés ou non et siliconés ou non.

Des exemples de polyuréthanes utiles incluent ceux divulgués dans les brevets EP 0 751 162, EP 0 637 600, FR 2 743 297, EP 0 648 485, EP 0 656 021, WO 94/03510 et EP 0 619 111.

Dans un autre mode de mise en oeuvre, les polymères peuvent être utilisés sous forme solubilisée ou peuvent être sous forme de dispersions de particules solides ou liquides (latex ou pseudolatex).

Selon un mode de mise en oeuvre plus préférentiel, ledit constituant additionnel est un tensioactif choisi parmi les tensioactifs anioniques, cationiques, amphotères et non ioniques.

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffinesulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycosidesulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyllactylates dont le groupe acyle comporte de 8 à 20 atomes de carbone.

En outre, on peut encore citer les acides alkyl-D-galactosideuroniques et leurs sels ainsi que les acides (alkyl en C₆₋₂₄)éthercarboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂ₒ)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (A) et (B) :

Rₐ-CONHCH₂CH₂-N(R_{b})(R_{c})(CH₂COO⁻) (A)

dans laquelle:
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   Rₐ'-CONHCH₂CH₂-N(B)(B') (B)
dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Dans une variante préférée, la composition selon l'invention comprend un ou plusieurs tensioactifs anioniques et un ou plusieurs tensioactifs amphotères ou zwittérioniques.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Le ou les agents tensioactifs utilisé(s) dans la composition selon l'invention peut ou peuvent être présents en une quantité allant de 0,1 % à 60 % en poids, par exemple de 1 % à 40 %, en outre de 5 % à 30 %, par rapport au poids total de la composition.

Selon un mode de réalisation, la composition selon l'invention comprend un ou plusieurs agents conditionneurs et un ou plusieurs tensioactifs.

De préférence, la composition selon l'invention comprend un ou plusieurs polymères cationiques et un ou plusieurs tensioactifs.

Selon un mode de mise en oeuvre particulier, la composition est un shampooing dans lequel un ou plusieurs tensioactifs est ou sont présents en une quantité allant de 4 % à 30 % par rapport au poids total de la composition.

Plus particulièrement, le shampooing peut également comprendre un ou plusieurs agents conditionneurs choisis parmi les tensioactifs cationiques et les polymères cationiques.

De préférence, le shampooing peut comprendre un ou plusieurs polymères cationiques.

Selon un autre mode de mise en oeuvre, la composition est un après-shampooing dans lequel un ou plusieurs tensioactifs cationiques et/ou un ou plusieurs polymères cationiques sont présents en une quantité allant de 0,1 % à 15 % par rapport au poids total de la composition et de préférence de 0,5 à 5% en poids.

La composition selon l'invention peut être vaporisable, par exemple par une pompe, ou peut être une composition aérosol sous pression. Elle peut être vaporisable par une valve de distribution contrôlée par une tête de distribution, qui comprend à son tour une buse, qui vaporise la composition aérosol. Une composition vaporisable selon l'invention comprend un solvant approprié. De manière avantageuse, le solvant approprié comprend au moins un solvant choisi parmi l'eau et des alcools inférieurs. Conformément à l'invention, on entend par alcool inférieur un alcool aliphatique en C₁ à C₄, tel que l'éthanol.

Lorsque la composition vaporisable selon l'invention est une composition aérosol, elle comprend en outre une quantité appropriée d'agent propulseur. L'agent propulseur comprend des gaz comprimés ou liquéfiés, qui sont normalement utilisés pour la préparation de compositions aérosol. Les gaz convenables incluent l'air comprimé, le gaz carbonique, l'azote, et les gaz susceptibles d'être solubles dans la composition, tels que l'éther diméthylique, les hydrocarbures fluorés ou non fluorés, et leurs mélanges.

La présente invention vise en outre un procédé de traitement de fibres kératiniques, notamment les cheveux, dans lequel la composition de coiffage repositionnable selon l'invention, telle que décrite ci-dessus, est appliquée sur les cheveux avant, pendant ou après la mise en forme de la coiffure.

La présente invention vise en outre l'utilisation d'une composition telle que décrite ci-dessus dans une formulation cosmétique de coiffage repositionnable ou pour sa préparation.

La présente invention vise également l'utilisation d'une composition telle que décrite ci-dessus comme shampooing ou après-shampooing.

La détermination de la capacité d'une composition contenant un copolymère (méth)acrylique (i) selon l'invention à donner un effet repositionnable peut être déterminée par un test in vivo.

Lorsque la composition est sous forme de lotion, par exemple, le test in vivo se déroule comme suit. Les cheveux du modèle sont lavés, puis divisés en deux portions symétriques, les côtés droit et gauche. La composition est appliquée sur un côté de la tête du modèle, puis rincée, alors qu'une composition de référence est appliquée sur l'autre côté de la tête. La composition de référence peut être choisie, par exemple, parmi l'eau, un produit commercial existant, ou une autre composition à l'étude. Le coiffeur sèche et coiffe les deux côtés de la tête. Les deux côtés de la tête sont évalués séparément pour l'effet de coiffage, les propriétés cosmétiques, et l'effet repositionnable. Par exemple, une fois séchés, les cheveux sont brossés dans différentes directions pour enlever la coiffure d'origine. Les cheveux sont alors brossés pour restaurer la coiffure d'origine. Le procédé pour enlever la coiffure, restaurer la coiffure, et évaluer le succès de restauration de la coiffure est répété au moins une fois de plus pour déterminer si la composition est une composition de coiffage repositionnable. Une composition de coiffage repositionnable permet (1) la restauration de la coiffure d'origine après brossage et (2) la création d'une nouvelle coiffure après brossage, qui peut également être restaurée après brossage. Si la composition à évaluer est sous une autre forme, telle qu'un shampoing ou un conditionneur, le test in vivo peut être modifié de façon appropriée par l'homme de l'art.

Il est entendu que l'homme de l'art pourrait reconnaître que ce n'est pas toutes les formulations qui donneraient un effet repositionnable pour tous les types de cheveu pendant l'essai in vivo et saura comment formuler et évaluer des compositions de coiffage repositionnables en vue des différents paramètres capillaires, tels que la longueur (court contre long), le diamètre (fin contre épais), la structure (frisé contre défrisé), l'état (gras, sec ou normal) ; et si les cheveux sont colorés, décolorés, permanentés ou défrisés. Ainsi, l'essai in vivo peut nécessiter l'essai sur 10-20 individus différents.

### EXEMPLES :

### 1)Préparation d'une émulsion de copolymère (méth)acrylique :

Un mélange de 300 grammes d'acrylate de 2-éthylhexyle (2-EHA), 175 grammes d'acrylate de n-butyle (BA), et 25 grammes de méthacrylate de 2-hydroxyéthyle (HEMA) a été préparé, donnant 500 grammes d'une solution de monomère contenant 60/35/5 parties de 2-EHA/BA/HEMA.

On a chargé 50 grammes de l'ensemble de la solution de monomère dans une fiole à résine divisée de deux litres ainsi que 380 grammes d'eau désionisée et 0,5 gramme de RHODACAL DS-10 (tensioactif dodécylbenzènesulfonate de sodium disponible dans le commerce auprès de Rhodia Chimie). La tête a été placée sur la fiole et on a attaché un thermocouple, une entrée d'azote et un agitateur mécanique. Le contenu a été chauffé par des lampes à infrarouge jusqu'à environ 60°C tout en agitant à 350 t/min. On a chargé une solution de 1 gramme d'amorceur persulfate de potassium dans 20 grammes d'eau désionisée, on a fermé la fiole de manière étanche, et on a fait un vide dans la fiole quatre fois, en le cassant à chaque fois avec de l'azote. La fiole a été maintenue à 60°C pendant 20 minutes, puis chauffée à 80°C pendant 10 minutes pour donner un polymère amorce. On a préparé une pré-émulsion à partir des 450 grammes restants de la solution de monomère en la chargeant d'une solution de 4,5 grammes de dodécylbenzènesulfonate de sodium dans 211 grammes d'eau désionisée et en agitant sous l'azote. Cette pré-émulsion a été ajoutée goutte à goutte à la fiole à résine divisée de deux litres contenant le polymère amorce à un débit de 6 grammes par minute. L'addition a duré presque 2 heures. Après l'addition, la vitesse d'agitation a été réduite à 200 t/min et la réaction a été maintenue à 80°C pendant deux heures, puis le latex résultant a été filtré sur une toile de fromagerie doublée dans un bocal. On a noté des taux faibles de coagulum autour du thermocouple et de la pale d'agitation.

### 2) Préparation d'une composition de coiffage :

On a préparé une composition de coiffage A selon l'invention sous forme de shampooing à partir des ingrédients indiqués ci-dessous. Les quantités de matières premières sont indiquées en % en l'état.

### Composition A

| | |
|---|---|
| Copolymère acrylate de 2 éthylhexyle (EMA) / | |
| acrylate de n-butyle (BA) / | |
| méthacrylate de 2-hydroxyéthyle (HEMA) 60/35/5 | |
| à 44,5% dans l'eau ⁽¹⁾ | 0,7% |
| Chlorure de poly-diméthyldiallylammonium | |
| dans l'eau à 40 % non stabilisé ⁽²⁾ | 0,25% |
| Polymère acrylique à 30 % en émulsion ⁽³⁾ | 3 % |
| Distérate d'éthylène glycol | 0,99% |
| Hydroxyde de sodium pur | 0,31% |
| Chlorure de sodium | 2 % |
| Lauryl éther sulfate de sodium (2,2 OE) | |
| à 70 % en solution aqueuse (72 %MA) | 14,11 % |
| Acide lauryléthercarboxylique (4,5 OE) | |
| à 90 % en solution aqueuse ⁽⁴⁾ | 2 % |
| Cocoyl bétaïne | |
| en solution aqueuse (38% MA) | 5,2 % |
| N-cocoyl amidoéthyl, | |
| N-éthoxycarboxy-méthyl glycinate de sodium | |
| à 31 % dans l'eau ⁽⁵⁾ | 1 % |
| Hexylène glycol (2 méthyl-2,4-pentanediol) | 1 % |
| Silicone aminée en émulsion aqueuse à 57,5% ⁽⁶⁾ | 2,7% |
| Conservateurs | qsp |
| Acide citrique | qs pH 6 |
| Parfum | qsp |
| Eau désionisée qsp | 100 % |

| | |
|---|---|
| ⁽¹⁾ vendu sous la dénomination CPC-00960-ACP45 Acrylates Copolymer par la société 3M ⁽²⁾ vendu sous la dénomination MERQUAT 100 par la société NALCO ⁽³⁾ vendu sous la dénomination AQUA SF1 par la société NOVEON ⁽⁴⁾ vendu sous la dénomination AKYPO RLM 45 par la société KAO ⁽⁵⁾ vendu sous la dénomination MIRANOL CON2. par la société RHODIA CHIMIE ⁽⁶⁾ vendu sous la dénomination DC2-8299 par la société DOW CORNING | |

La composition A utilisée sous forme de shampooing, c'est-à-dire appliquée sur les cheveux, puis lavée et rincée, confère aux cheveux une bonne facilitée de coiffage, un effet repositionnable.

On obtient des cheveux lisses qui présentent de bonnes propriétés de démêlage, de douceur et de brillance.

On a préparé une composition de coiffage B selon l'invention sous forme de shampooing à partir des ingrédients indiqués ci-dessous. Les quantités de matières premières sont indiquées en % en l'état.

### Composition B

| | |
|---|---|
| Copolymère acrylate de 2 éthylhexyle (EMA) / | |
| acrylate de n-butyle (BA) / | |
| méthacrylate de 2-hydroxyéthyle (HEMA) 60/35/5 | |
| à 44,5 % dans l'eau ⁽¹⁾ | 0,7% |
| Chlorure de poly-diméthyldiallylammonium | |
| dans l'eau à 40 % non stabilisé ⁽²⁾ | 0,25% |
| Polymère acrylique à 30 % en émulsion ⁽³⁾ | 3 % |
| Distérate d'éthylène glycol | 1% |
| Hydroxyde de sodium pur | 0,31% |
| Chlorure de sodium | 2 % |
| Lauryl éther sulfate de sodium (2.2 OE) | |
| à 70 % en solution aqueuse (72 %MA) | 14,11 % |
| Acide lauryléthercarboxylique (4,5 OE) | |
| à 90% en solution aqueuse ⁽⁴⁾ | 2 % |
| Cocoyl bétaïne | |
| en solution aqueuse (38% MA) | 5,2 % |
| N-cocoyl amidoéthyl, | |
| N-éthoxycarboxy-méthyl glycinate de sodium | |
| à 31 % dans l'eau ⁽⁵⁾ | 1 % |
| Hexylène glycol (2 méthyl-2,4-pentanediol) | 1 % |
| Silicone à 54% dans un mélange | |
| de solvant non aqueux ⁽⁶⁾ | 2,7% |
| Conservateurs | qsp |
| Acide citrique | qs pH 6 |
| Parfum | qsp |
| Eau désionisée qsp | 100 % |

| | |
|---|---|
| ⁽¹⁾ vendu sous la dénomination CPC-00960-ACP45 Acrylates Copolymer par la société 3M ⁽²⁾ vendu sous la dénomination MERQUAT 100 par la société NALCO ⁽⁴⁾ vendu sous la dénomination AQUA SF1 par la société NOVEON ⁽⁴⁾ vendu sous la dénomination AKYPO RLM 45 par la société KAO ⁽⁵⁾ vendu sous la dénomination MIRANOL CON2. par la société RHODIA CHIMIE ⁽⁶⁾ vendu sous la dénomination DC 190 Fluid par la société DOW CORNING | |

La composition B utilisée sous forme de shampooing, c'est-à-dire appliquée sur les cheveux, puis lavée et rincée, confère aux cheveux une bonne facilitée de coiffage, un effet repositionnable.

On obtient des cheveux lisses qui présentent de bonnes propriétés de démêlage, de douceur et de brillance.

## Revendications

1. Composition rincée de coiffage repositionnable des fibres kératiniques, et plus particulièrement des cheveux, **caractérisée en ce qu'**elle comprend, dans un véhicule cosmétiquement acceptable :
(i) un ou plusieurs copolymères (méth)acryliques comprenant :
- (a) des motifs issus d'un ou de plusieurs monomères choisis parmi les monomères de (méth)acrylate de butyle, le ou les monomères (a) représentant de 15 à 50 % en poids par rapport à la quantité totale de monomères,
- (b) des motifs issus d'un ou de plusieurs monomères choisis parmi les monomères de (méth)acrylate d'hydroxyalkyle, et
- (c) des motifs éventuels issus d'un ou plusieurs monomères copolymérisables autres que lesdits monomères (a) et (b) ;
(ii)une ou plusieurs silicones.

2. Composition selon la revendication 1, **caractérisée en ce que** le monomère (a) est choisi parmi les (méth)acrylates de n-butyle, les (méth)acrylates d'isobutyle, les (méth)acrylates de t-butyle et les (méth)acrylates de 2-méthylbutyle.

3. Composition selon l'une quelconque des revendications précédentes, **caractérisée en ce que** le monomère (b) est choisi parmi les (méth)acrylates de 2-hydroxyéthyle et les (méth)acrylates d'hydroxypropyle.

4. Composition selon l'une quelconque des revendications précédentes, **caractérisée en ce que** le monomère (c) est choisi parmi les monomères de (méth)acrylate d'alkyle (i'), les monomères polaires (ii'), et les monomères polymérisables par voie radicalaire libre à insaturation éthylénique (iii').

5. Composition selon la revendication 4, **caractérisée en ce que** les monomères de (méth)acrylate d'alkyle (i') sont choisis parmi les (méth)acrylates de méthyle, les (méth)acrylates d'isobornyle, les (méth)acrylates d'éthyle, le (méth)acrylates d'iso-octyle et les (méth)acrylates de 2-éthylhexyle.

6. Composition selon la revendication 4, **caractérisée en ce que** les monomères polaires (ii') sont choisis parmi les acides (méth)acryliques, les acides itaconiques, les N-vinylpyrrolidones, les N-vinylcaprolactames, les (méth)acrylamides substitués, les (méth)acrylates de diméthylaminoéthyle, les acrylonitriles, les (méth)acrylates de 2-carboxyéthyle, les anhydrides maléiques, et les monoacrylates de méthoxypolyéthylèneglycol 550.

7. Composition selon la revendication 4, **caractérisée en ce que** les monomères polymérisables par voie radicalaire libre à insaturation éthylénique (iii') sont choisis parmi le styrène et les esters de vinyle en C₁-C₄.

8. Composition selon l'une quelconque des revendications précédentes, **caractérisée en ce que** le copolymère (méth)acrylique (i) comprend :
(a) de 30 à 40 % en poids de motifs issus d'au moins un monomère choisi parmi les monomères d'acrylate de n-butyle,
(b) de 2 à 10 % en poids de motifs issus d'au moins un monomère choisi parmi les monomères de (méth)acrylate de 2-hydroxyéthyle, et
(c) de 50 à 70 % en poids de motifs issus d'au moins un monomère d'acrylate de 2-éthylhexyle.

9. Composition cosmétique selon l'une quelconque des revendications précédentes, **caractérisée en ce que** la silicone est une silicone non volatile choisie parmi les polyorganosiloxanes insolubles dans la composition.

10. Composition cosmétique selon la revendication 9,
**caractérisée en ce que** les polyorganosiloxanes sont des polyorganosiloxanes non volatils choisis parmi les polyalkylsiloxanes, des polyarylsiloxanes, des polyalkylarylsiloxanes, des gommes et résines de silicones, des polyorganosiloxanes modifiés par des groupements organofonctionnels ainsi que leurs mélanges.

11. Composition cosmétique selon la revendication 10,
**caractérisée par le fait que**
(a) les polyalkylsiloxanes sont choisi parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisi parmi :
- les polydiméthylméthylphénylsiloxanes, les polydiméthyl diphénylsiloxanes linéaires et/ou ramifiés de viscosité de 1.10⁻⁵ à 5.10⁻² m²/s à 25°C.
(c) les gommes de silicone sont choisies parmi les polydiorganosiloxanes ayant des masses moléculaires moyennes en nombre comprises entre 200 000 et 1000000 utilisés seuls ou sous forme de mélange dans un solvant ;
(d) les résines sont choisies parmi les résines constituées d'unités : R₃SiO_{1/2}, R₂SiO_{2/2}, RSiO_{3/2}, SiO_{4/2}
dans lesquelles R représente un groupement hydrocarboné possédant de 1 à 16 atomes de carbone ou un groupement phényle ;
(e) les silicones organomodifiées sont choisies parmi les silicones comportant dans leur structure un ou plusieurs groupements organofonctionnels fixés par l'intermédiaire d'un radical hydrocarboné.

12. Composition cosmétique selon l'une quelconque des revendications précédentes, **caractérisée en ce que** la silicone est une silicone aminée.

13. Composition selon l'une quelconque des revendications précédentes, **caractérisée en ce qu'**elle comprend en outre un ou plusieurs tensioactifs choisis parmi les tensioactifs anioniques, cationiques, amphotères, non-ioniques et leurs mélanges.

14. Procédé de traitement cosmétique des cheveux, comprenant l'application sur les cheveux, avant leur mise en forme, d'une composition selon l'une quelconque des revendications 1 à 13.

15. Procédé de traitement des cheveux, comprenant :
(1) l'application sur les cheveux, avant la mise en forme initiale de la coiffure, d'une composition selon l'une quelconque des revendications 1 à 13, puis
(2) la mise en forme de la coiffure au moins une fois, **caractérisé en ce qu'**une composition supplémentaire ou de la chaleur supplémentaire n'est pas nécessaire.
